(19) Europäisches Patentamt
European Patent Office
Office européen des brevets

(11) Publication number: 0 147 883
A2

(12) # EUROPEAN PATENT APPLICATION

(21) Application number: 84201801.2

(22) Date of filing: 05.12.84

(51) Int. Cl.4: A 61 K 31/425
A 61 K 47/00

(30) Priority: 21.12.83 US 564122

(43) Date of publication of application:
10.07.85 Bulletin 85/28

(84) Designated Contracting States:
AT BE CH DE FR GB IT LI LU NL SE

(71) Applicant: JANSSEN PHARMACEUTICA N.V.
Turnhoutsebaan 30
B-2340 Beerse(BE)

(72) Inventor: Van der Veken, Guido Jozef Louisa
Krombrakenstraat 9
B-2460 Kasterlee(BE)

(72) Inventor: Ligtvoet, Theo Frans Maria Cornelius
Antwerpseweg 169
B-2151 Vlimmeren(BE)

(54) **Aqueous non-aggressive anthelmintic pour-on formulations.**

(57) A spot-on formulation for combatting helmintic infestations in non-human animals containing per 100 ml from 0.04 to 0.15 moles of a pharmaceutically acceptable acid addition salt of tetramisole or levamisole and a vehicle consisting of from 1 to 60% v/v of a penetration enhancing and/or moistening agent in water.

# AQUEOUS NON-AGGRESSIVE ANTHELMINTIC POUR-ON FORMULATIONS.

The present invention is concerned with aqueous pour-on formulations for combatting helmintic infestations in non-human animals.

The method of systemically administering drugs by pouring or spraying a composition, comprising the desired drug, onto any part of the skin is generally known in veterinary medicine as the pour-on method. Drugs, administered following this method, are absorbed by the skin and, after they have penetrated through the skin, they are transmitted systemically within the animal [see, for example, W.M. Rogoff and P.H. Kohler, J. Econ. Ent., 53, 814-817 (1960) and B. Idson, J. Pharm. Sci.,64, 901-924 (1975)].

In comparison with the parenteral administration methods the pour-on method offers distinct advantages. For example, there is no need to held the animal, sterile precautions are not necessary and especially trained staff is not required.

Furthermore, compared with the oral administration methods, the pour-on method has the advantage that each animal receives an exactly defined amount of the desired drug.

Tetramisole, being chemically designated as 2,3,5,6-tetrahydro-6-phenylimidazo[2,1-b]thiazole and the laevo isomer thereof, levamisole, have been described in U.S. Patent Nos. 3,274,209,

respectively 3,463,786.

The compounds are powerful anthelmintic agents. Structurally, tetramisole is represented by the formula

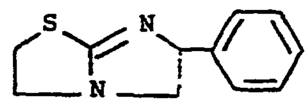

(I)

Anthelmintic pour-on compositions which contain tetramisole or levamisole have been described in U.S. Patent Nos. 4,070,476 and 3,980,791. Said compositions have the disadvantage that the carriers, which are the most effective for helping the penetration of the anthelmintic through the skin, are at the same time the most aggressive to the treated skin, resulting in, for example, subcutaneous bleedings, necrosis, hair and skin diseases and, at worst, open wounds [severe skin irritations, caused by pour-on compositions, are described, e.g., in Veterinär Medizinische Nachrichten 1978 (1), 109-112)].

Tetramisole and/or levamisole containing pour-on compositions having a strongly reduced aggressivity to the treated skin have also been described in U.S. Patent No. 4,278,685 and in European Patent No. 61,806. However, these formulations have some less advantageous properties originating from their organic nature such as, for example, their expense combined with less advantageous influences on the environment and the safety and health of the applicator.

Additionally, these organic formulations, having good penetration properties for cattle-skin, are less effective for penetrating through the skin of sheep.

An additional limitation of the hereinabove-mentioned organic formulations is their limited solubility of polar compounds in general, and, more particularly, of the acid addition salts of tetramisole and/or levamisole.

Additionally, as water is taught to be ineffective as a liquid carrier for passing the compound through the skin of an animal (See, for example, U.K. Patent No. 1,464,552) useful aqueous pour-on formulations for combatting helmintic infestations in non-human

animals are hitherto unknown.

The present invention is concerned with aqueous pour-on formulations for combatting helmintic infestations in non-human animals containing besides a vehicle from 0.04 to 0.15 moles of tetramisole or levamisole per 100 ml wherein the tetramisole or levamisole is present in the form of a pharmaceutically acceptable acid addition salt and the vehicle consists of from 1 to 60 v/v % of a penetration enhancing and/or moistening agent in water.

Aqueous pour-on formulations wherein the vehicle consists of from 10 to 60 v/v % of a penetration enhancing and/or moistening agent in water are particularly preferred due to their excellent spreading properties.

Vehicles which combine an effective penetration enhancing property with a suitable moistening effect contain, for example, lanolin and woolwax derivatives, e.g. Amerlate P®, Acetulan®, Aqualose LL100®, Aqualose DL12®, Aqualose SLW®, Lanesta L® and the like; amido ether sulfate complexes; sulfonated aliphatic polyesters, e.g. Humifen®; esters, eg., 2-propyl myristate, 2-propyl palmilate, alkoxylated myristyl propionate, lauric acid hexyl ester, caprionic acid tri- glycerides and the like, addition products of 1 to 60 moles of ethylene oxide with 1 mole of a phenol which is further substituted with at least one $C_1$-$C_{15}$ alkyl group, e.g. Cemulsol NP10® and the like; addition products of 1 to 60 moles of ethylene oxide with 1 mole of ricinus oil, e.g, Soprophor B®, Heliwet EO33 and the like; esters of hydroxy-substituted carboxylic acids, e.g. Polysolvan O®; alcohols, e.g. 2-propanol, 2-butoxyethanol, 2-(2-butoxyethoxy)ethanol; 1,2,6-trihydroxyhexane, 2-methyl-2,4-pentanediol and the like; donor-acceptor solvents, e.g. dimethyl sulfoxide (DMSO), N,N-dimethylformamide (DMF), N,N-dimethylacetamide (DMA), N-methylpyrrolidone (NMP) and the like; 4-hydroxy-4-methyl-2-pentanone; $C_1$-$C_6$-alkyl esters of adipic-, glutaric and succinic acid; and mixtures thereof.

Although the above-mentioned pour-on formulations are, as such, very suitable for percutaneous administration, said vehicle may possibly contain additives of any nature in minor proportions, which

additives do not introduce a significant deleterious effect on the skin. Said additives may facilitate the administration to the skin of the animals and/or may be helpful for preparing the desired formulations. The concerned additives may also consist of substances the taste of which deters animals from licking the applied formulations off the animals treated, pigments making it possible to recognize the animals, conservating agents, defoaming agents, antioxidants and the like.

Besides a pharmaceutically acceptable acid addition salt of tetramisole or levamisole and the vehicle the formulations of the present invention may also contain other pharmaceutically active compounds such as, for example, other substances with anthelmintic and/or insecticidal properties.

Suitable acid addition salts of tetramisole or levamisole are those formed by treating tetramisole or levamisole with appropriate acids such as, for example, inorganic acids such as hydrohalic acids, e.g. hydrochloride, hydrobromic and the like, sulfuric acid, nitric acid, phosphoric acid and the like; or organic acids such as, for example, acetic, propanoic, hydroxyacetic; 2-hydroxypropanoic, 2-oxo-propanoic, propanedioic, butanedioic, (Z)-2- butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, $C_1$-$C_{18}$alkylmethylbenzenesulfonic, cyclohexanesulfonic, 2-hydroxybenzoic, 4-amino-2-hydroxybenzoic, salicyclic and the like acids.

Since the anthelmintic activity of tetramisole is exerted essentially by the laevo isomer, in a preferred embodiment acid-addition salts of levamisole are employed as the anthelmintically active agent.

The formulations of the present invention combine a good stability and a desirable percutaneous absorption with the advantages of aqueous mediums such as, for example, a high flash point and the absence of disadvantageous influences on the environment and the safety of the applicator.

Additionally, these aqueous formulations have not only good penetration properties for cattle-skin, but contrary to art-known formulations the subject pour-on formulations have properties which make them very suitable for the percutaneous absorption of the skin of any warm-blooded animals, especially for the percutaneous absorption of sheep skin and cattle skin, without causing any appreciable skin-irritation.

The formulations of the present invention may be prepared by mixing an acid addition salt of tetramisole or levamisole with water, if desired at elevated temperatures, subsequently adding the moistening and penetration enhancing agent and, if desired, suitable additives.

It is evident that the acid addition salts of tetramisole and/or levamisole may also be prepared in situ by mixing tetramisole and/or levamisole with a suitable amount of an appropriate acid.

The efficacy of the concerned anthelmintic pour-on formulations can be illustrated by the eggs-per-gram count technique conducted on faeces samples taken before and after treatment of sheep.

<u>Experimental part</u>

The following examples are intended to illustrate and not to limit the scope of the present invention. Unless otherwise stated all parts therein are by weight.

In the following examples Cemulson NP10® is a Trade Mark of a mixture of addition products of nonylphenols with ethylene oxide, wherein an average of 10 moles of ethylene oxide has been reacted with 1 mole of nonylphenol; Soprophor B® is a Trade Mark of a mixture of addition products of ricinus oil with ethylene oxide; Aqualose LL100® is a Trade Mark of a polyoxyalkylene co-condensate of lanolin oil with mixed ethylene and propylene oxides, the mixed chains averaging 100 units; Aqualose SLW® is a homogeneous blend containing 20% by weight of natural lanolin oil with ethoxylated alcohols derived entirely from lanolin and having a mean chain length of 20 ethylene oxide units; Heliwet EO33 is a Trade Mark of polyethoxylated ricinus oil; Estasol® is a Trade Mark of a mixture of 20% dimethyl adipate, 60% dimethyl glutarate and 20% dimethyl succinate; Atplus 300F® is a Trade Mark of a mixture of surfactants and an anionic wetting agent; Aqualose DL12® is

a Trade Mark of a mixture of ethoxylated lanolin derivatives; Aqualose AH® is a Trade Mark of a mixture of ethoxylated lanolin derivatives; Polysolvan O® is a Trade Mark of butyl hydroxy acetic acid ester; and Lanesta L® is a mixture derived from the isopropyl esters of the natural fatty acids occurring in pharmaceutical anhydrous lanolin.

A. Preparation of formulations

Example I:

Formulation 1: 15.4% w/v levamisole hydroxyacetic acid salt, 10% w/v Cemulsol NP10®, and aqua dest. ad 100%.

Preparation:

10 Parts of levamisole were added to a mixture containing 5.4 parts of hydroxyacetic acid in 50 parts of water. After solubilization 10 parts of Cemulsol NP10® were added and the mixture was diluted with water ad 100 ml while stirring.

Example II:

Formulation 2: 15.4% w/v levamisole hydroxyacetic acid salt, 10% w/v Cemulsol NP10®, 20% v/v 2-propanol, and aqua dest. ad 100%.

Preparation:

10 Parts of levamisole were added to a mixture containing 5.4 parts of hydroxyacetic acid in 40 parts of water. After solubilization 10 parts of Cemulsol NP10® and 20 ml of 2-propanol were added and the mixture was diluted with water ad 100 ml while stirring.

Example III:

Following the preparation-procedure described in example II the following formulations were also prepared:

Formulation 3: 15.4% w/v levamisole hydroxyacetic acid salt, 10% w/v Cemulsol NP10®, 20% v/v dimethylsulfoxide, and aqua dest. ad 100%.

Formulation 4: 15.4% w/v levamisole hydroxyacetic acid salt, 10% w/v Soprophor B®, 20% v/v 2-propanol, and aqua dest. ad

100%.

Formulation 5: 31.2% w/v levamisole hydroxyacetic acid salt, 25% w/v Cemulsol NP10®, 10% w/v 2-butoxyethanol, 4% v/v Lanesta L® and aqua dest. ad 100%.

Example IV:

Formulation 6: 11.8% w/v levamisole hydrochloric acid salt, 10% w/v Cemulsol NP10®, 20% v/v 2-propanol, and aqua dest. ad 100%.

Preparation:

11.8 Parts of levamisole hydrochloric acid salt were added to 40 parts of water. After solubilization 10 parts of Cemulsol NP10® and 20 ml of 2-propanol were added and the mixture was diluted with water ad 100 ml while stirring.

Example V:

Following the preparation-procedure described in example IV the following formulations were also prepared:

Formulation 7: 23.6% w/v levamisole hydrochloric acid salt, 25% w/v Cemulsol NP10®, 10% v/v 2-butoxyethanol, 4% Lanesta L®, and aqua dest. ad 100%.

Formulation 8: 11.8% w/v levamisole hydrochloric acid salt, 25% w/v Cemulsol NP10®, 20% v/v 2-(2-butoxyethoxy)ethanol 5% Lanesta L®, and aqua dest. ad 100%.

Formulation 9: 11.8% w/v levamisole-hydrochloric acid salt, 25% w/v Cemulsol NP10®, 10% v/v 2-(2-butoxyethoxy)-ethanol 4% v/v Lanesta L®, and aqua dest. ad 100%.

Formulation 10: 11.8% w/v levamisole hydrochloric acid salt, 25% w/v Aqualose LL100®, 10% v/v 2-(2-butoxyethoxy)ethanol, and aqua dest. ad 100%.

Formulation 11: 11.8% w/v levamisole hydrochloric acid salt, 25% w/v Aqualose SLW®, and aqua dest. ad 100%.

Formulation 12: 11.8% w/v levamisole hydrochloric acid salt, 25% w/v Aqualose LL100®, and aqua dest. ad 100%.

Formulation 13: 11.8% w/v levamisole hydrochloric acid salt, 25% w/v

Cemulsol NP10® 10% v/v 2-butoxyethanol and 4% v/v Lanesta L®, and aqua dest. ad 100%.

Formulation 14: 11.8% w/v levamisole hydrochloric acid salt, 25% w/v Cemulsol NP10®, 10% v/v 2-propanol 4% v/v Lanesta L® and aqua dest. ad 100%.

Formulation 15: 23.6% w/v levamisole hydrochloride, 25% w/v Cemulsol NP10®, 10% v/v 2-propanol, 4% v/v Lanesta L® and aqua dest. ad 100%.

Formulation 16: 20% w/v levamisole hydrochloride, 25% w/v Cemulsol NP10®, 10% v/v 2-propanol, 4% v/v Lanesta L® and aqua dest. ad 100%.

Formulation 17: 11.8% w/v levamisole hydrochloride, 20% w/v Heliwet EO33 and aqua dest. ad 100%.

Formulation 18: 11.8% w/v levamisole hydrochloride, 20% w/v Cemulsol NP10® and aqua dest. ad 100%.

Formulation 19: 11.8% w/v levamisole hydrochloride, 20% w/v Aqualose DL12® and aqua dest. ad 100%.

Formulation 20: 11.8% w/v levamisole hydrochloride, 20% w/v Cemulsol NP10®, 10% v/v NMP and aqua dest. ad 100%.

Formulation 21: 11.8% w/v levamisole hydrochloride, 20% w/v Cemulsol NP10®, 10% v/v DMSO and aqua dest. ad 100%.

Formulation 22: 11.8% w/v levamisole hydrochloride, 20% w/v Cemulsol NP10®, 10% Estasol® and aqua dest. ad 100%.

Formulation 23: 10.0% w/v levamisole base, 9.6% w/v citric acid, 0.5% v/v Atplus 300F®, 20% v/v diacetone alcohol, 10% v/v Cemulsol NP10® and aqua dest. ad. 100%.

Formulation 24: 10.0% w/v levamisole base, 9.6% w/v citric acid, 20% v/v Cemulsol NP10®, 10% v/v 2-propanol, 4% v/v Lanesta L® and aqua dest. ad. 100%.

Formulation 25: 10.0% w/v levamisole base, 9.6% w/v citric acid, 20% w/v Aqualose DL12®, 20% v/v diacetone alcohol and aqua dest. ad. 100%.

Formulation 26: 10.0% w/v levamisole base, 9.6% w/v citric acid, 20% w/v Aqualose DL12®, 20% v/v NMP and aqua dest. ad. 100%.

Formulation 27: 11.8% w/v levamisole hydrochloride, 20% v/v NMP, 20% w/v

Aqualose DL12® and aqua dest. ad 100%.

Formulation 28: 11.8% w/v levamisole hydrochloride, 20% v/v NMP, 20% w/v Cemulsol NP10® and aqua dest. ad 100%.

Formulation 29: 11.8% w/v levamisole hydrochloride, 30% v/v NMP, 15% w/v Aqualose DL12® and aqua dest. ad 100%.

Formulation 30: 11.8% w/v levamisole hydrochloride, 30% v/v NMP, 10% w/v Aqualose DL12® and aqua dest. ad 100%.

Formulation 31: 11.8% w/v levamisole hydrochloride, 20% v/v NMP, 7.5% w/v Aqualose DL12® and aqua dest. ad 100%.

Formulation 32: 11.8% w/v levamisole hydrochloride, 30% v/v NMP, 5.0% w/v Aqualose DL12® and aqua dest. ad 100%.

Formulation 33: 11.8% w/v levamisole hydrochloride, 10% v/v NMP, 10% w/v Aqualose DL12® and aqua dest. ad 100%.

Formulation 34: 10% w/v levamisole base, 5.4% w/v glycolic acid 70%, 15% v/v NMP, 10% w/v Aqualose DL12® and aqua dest. ad 100%.

Formulation 35: 10% w/v levamisole base, 5.4% w/v glycolic acid 70%, 10% w/v Aqualose LL100®, 10% v/v NMP and aqua dest. ad 100%.

Formulation 36: 10% w/v levamisole base, 15.7% w/v dobanic acid, 10% v/v NMP, 10% w/v Aqualose LL100® and aqua dest. ad 100%.

Formulation 37: 10% w/v levamisole base, 6.8% w/v salicylic acid, 20% v/v NMP, 10% w/v Aqualose DL12® and aqua dest. ad 100%.

Formulation 38: 10% w/v levamisole base, 6.8% w/v salicylic acid, 10% v/v NMP, 10% w/v Aqualose LL100® and aqua dest. ad 100%.

Formulation 39: 10% w/v levamisole base, 5% v/v acetic acid, 10% w/v Aqualose DL12® and aqua dest. ad 100%.

Formulation 40: 10% w/v levamisole base, 5% acetic acid, 20% v/v NMP, 10% v/v Aqualose LL100® and aqua dest. ad 100%.

Formulation 41: 10% w/v levamisole base, 5.4% w/v glycolic acid 70%, 50% v/v NMP, 10% w/v Aqualose DL12® and aqua dest. ad 100%.

Formulation 42: 10% w/v levamisole base, 5% w/v o-phosphoric acid 98%, 10% w/v Aqualose DL12®, 50% v/v NMP and aqua dest. ad 100%.

Formulation 43: 11.8% w/v levamisole hydrochloride, 50% v/v ethanol and aqua dest. ad 100%.

Formulation 44: 10% w/v levamisole base, 5.4% w/v glycolic acid 70%, 50%

v/v ethanol and aqua dest. ad 100%.

Formulation 45: 10% w/v levamisole base, 5% w/v o-phosphoric acid 98%, 50% v/v ethanol and aqua dest. ad 100%.

Formulation 46: 10% w/v levamisole base, 5.4% w/v glycolic acid 70%, 50% v/v butyldioxitol and aqua dest. ad 100%.

Formulation 47: 10% w/v levamisole base, 5% w/v o-phosphoric acid 98%, 10% w/v Aqualose LL100®, 10% v/v NMP and aqua dest. ad 100%.

Formulation 48: 10% w/v levamisole base, 10% Aqualose DL12®, 40% v/v butyldioxitol, 10% v/v Shellsol AB, 5.4% w/v glycolic acid 70%, and aqua dest. ad 100%.

Formulation 49: 11.8% w/v levamisole hydrochloride, 40% v/v trihydroxyhexane, 10% w/v Aqualose LL100®, and aqua dest. ad 100%.

Formulation 50: 10% w/v levamisole base, 5% acetic acid, 50% v/v butyldioxitol, and aqua dest. ad 100%.

Formulation 51: 11.8% w/v levamisole hydrochloride, 10% w/v Aqualose LL100®, 50% v/v butyldioxitol, and aqua dest. ad 100%.

Formulation 52: 11.8% w/v levamisole hydrochloride, 50% v/v Polysolvan O®, and aqua dest. ad 100%.

Formulation 53: 11.8% w/v levamisole hydrochloride, 10% w/v Aqualose LL100®, 50% v/v 2-propanol, and aqua dest. ad 100%.

Formulation 54: 11.8% w/v levamisole hydrochloride, 10% v/v ethanol, 20% w/v Aqualose AH®, and aqua dest. ad 100%.

Formulation 55: 11.8% w/v levamisole hydrochloride, 30% v/v 2-(2-butoxy-ethoxy)ethanol, 10% v/v N-methylpyrrolidone, 10% w/v Aqualose LL100®, and aqua dest. ad 100%.

Formulation 56: 11.8% w/v levamisole hydrochloride, 10% w/v Aqualose LL100®, 40% v/v 2-methyl-2,4-pentanediol, and aqua dest. ad 100%.

Example VI

Artificially infected sheep were treated by applying a formulation along the backline. Faeces were examined by routine Mc.Master technique, described in J. Counc. Sci. Industr. Res. Austr. 12, 50 (1939), before treatment and 7 days after treatment.

4 Weeks before treatment the sheep were artificially infected with L3-larvae of different worm species.

Prior to treatment sheep were weighed and the tested formulations were applied along the backline in such a quantity that 10 mg of levamisole/kg of body weight was administered.

Irritation-scores were noted on day 7 and day 14 after treatment. None of the formulations caused any detectable irritation.

Table 1: % Efficacy of a number of formulations 7 days after treatment.

| Formulations No. | % Efficacy |
|---|---|
| 1 | 93 |
| 3 | 100 |
| 5 | 100 |
| 7 | 96 |
| 8 | 100 |
| 14 | 100 |
| 29 | 95 |
| 30 | 100 |
| 31 | 100 |
| 33 | 100 |
| 35 | 100 |
| 39 | 190 |
| 44 | 92 |
| 46 | 100 |
| 48 | 100 |
| 50 | 100 |
| 51 | 91 |
| 53 | 93 |

CLAIMS

1. A spot-on formulation for combatting helmintic infestations in non-human animals, containing besides a vehicle from 0.04 to 0.15 moles of tetramisole or levamisole per 100 ml characterized in that the tetramisole or levamisole is present in the form of a pharmaceutically acceptable acid addition salt and the vehicle consists of from 1 to 60 % v/v of a penetration enhancing and/or moistening agent in water.

2. A pour-on formulation according to claim 1 wherein the vehicle consists of from 10 to 60% v/v of a penetration enhancing and/or moistening agent in water.